# EUROPEAN PATENT SPECIFICATION

(11) **EP 2 883 835 B1**
(45) Date of publication and mention of the grant of the patent: **15.07.2020**
(21) Application number: 13828194.4
(22) Date of filing: 06.03.2013
(51) Int. Cl.: C01B 32/05, C01B 32/15, C09K 11/65, B82Y 5/00

(54) **PREPARATION METHOD OF HETEROATOM DOPED MULTIFUNCTIONAL CARBON QUANTUM DOT**
VERFAHREN ZUR HERSTELLUNG VON HETEROATOM-DOTIERTEN MULTIFUNKTIONELLEN QUANTENPUNKTEN
PROCÉDÉ DE PRÉPARATION DE POINT QUANTIQUE DE CARBONE MULTIFONCTIONNEL DOPÉ PAR DES HÉTÉROATOMES

(30) Priority: 06.08.2012 CN 201210277190; 19.02.2013 CN 201310053616; 19.02.2013 CN 201310053661; 19.02.2013 CN 201310053636; 19.02.2013 CN 201310053831
(43) Date of publication of application: 17.06.2015
(73) Proprietor: Technical Institute of Physics and Chemistry of the Chinese Academy of Sciences, Haidian District Beijing 100190 (CN)
(72) Inventor: WANG, Pengfei, Beijing 100190 (CN); GE, Jiechao, Beijing 100190 (CN); LAN, Minhuan, Beijing 100190 (CN); LIU, Weimin, Beijing 100190 (CN)
(74) Representative: Serjeants LLP
(86) International application number: PCT/CN2013/072230
(87) International publication number: WO 2014/023097

(56) References cited:
- WO-A2-2008/060642
- CN-A- 102 180 459
- CN-A- 102 492 724
- CN-A- 102 897 745
- JP-A- 2007 234 962
- US-A1- 2010 025 662
- US-A1- 2012 178 099
- LARSON D R ET AL: "WATER-SOLUBLE QUANTUM DOTS FOR MULTIPHOTON FLUORESCENCE IMAGING IN VIVO", SCIENCE, AMERICAN ASSOCIATION FOR THE ADVANCEMENT OF SCIENCE, US, vol. 300, no. 5624, 1 January 2003 (2003-01-01), pages 1434-1436, XP008053308, ISSN: 0036-8075, DOI: 10.1126/SCIENCE.1083780

## Description

### BACKGROUND OF THE INVENTION

The present invention relates to a method for preparing heteroatom doped multifunctional carbon quantum dot and application thereof, particularly to a method for preparing heteroatom doped multifunctional carbon quantum dot and application thereof in fields of biomedicine, catalysts, photoelectric devices, etc.

### BACKGROUND

Carbon is the basis of all known life on the earth. Due to possessing the diverse characteristics of the electron orbit (sp1, sp2, sp3), carbon forms a large number of substances with peculiar structures and properties.

Carbon quantum dot is a new type of carbon material discovered in 2004. Compared to traditional semiconductor quantum dots and organic dyes, carbon quantum dot, as a new member of the carbon family, not only keeps the advantages of carbon materials such as low toxicity, good biocompatibility, but also has the extraordinary properties such as adjustable light-emitting range, large two-photon absorption cross section, good light stability, no scintillation, ease of functionalization, to be produced inexpensively and on a large scale, and it is expected to have broad application prospects in fields of optoelectronic devices, nano-catalysts, biomedicine, etc [Angew. Chem. Int. Ed, 2010, 49, 6726-6244; Chem. Comm. 2012, 48, 3686-3705; J. Mater. Chem., 2012, 22, 24230-24253; Energy Environ. Sci., 2012, 5, 8869-8890]. So far, rapid progress has been made on the preparation of carbon quantum dot and the studies on biomedicine, nano-catalysis, optoelectronic devices, etc, but the intrinsic disadvantages of carbon quantum dot, such as short emission wavelength, weak catalytic performance and low photoelectric conversion efficiency, restrict the popularization of carbon quantum dot in the field of practical applications. Thus there is a need to improve methods of preparation and surface modification, and continue to study the surface photoelectric properties of carbon quantum dot, especially the heteroatom doped carbon quantum dot. It is reported that the heteroatom doped carbon quantum dot may effectively modify the properties of the quantum dot including electronic properties and surface chemical properties [Energy Environ. Sci., 2012, 5, 8869-8890]. Now studies on the preparation and application of heteroatom doped carbon quantum dots are not common, and the doped heteroatoms are mainly the nitrogen atoms and oxygen atoms [J. Mater. Chem., 2012, 22, 16714-16718, Carbon, 2011, 49, 5207-5212]. Therefore, the exploration for the preparation and application of carbon quantum dots doped by heteroatom based on N, S, Si, Se, P, As, Ge, Gd, B, Sb, Te etc, will hopefully break the bottlenecks of practical application.

US 2010/025662 discloses a method for preparing a heteroatom doped multifunctional quantum dot comprising the steps of:
1) adding to a conjugated polymer, 0-1 M aqueous solution of acids or bases with the mass of 0.01-1000 times as many as the mass of the conjugated polymer, mixing uniformly and obtaining a reaction solution (oleic acid),
2) heating the reaction solution up to 115°C, and reacting for 30 minutes,
3) cooling after the reaction, collecting the reaction solution, separating and purifying to obtain heteroatom doped multifunctional carbon quantum dots (example 2).

US 2012/178099 discloses a method of synthesis of fluorescent carbon nanotubes comprising the steps of:
1) providing carbohydrate molecules,
2) carbonizing/degrading the carbohydrate, and
3) controlling the carbon growth conditions/rate of carbonization to obtain highly fluorescent nanoparticles of desired size and quantum yield by controlling nucleation-growth kinetics.

Larson D R et al. "Water-Soluble Quantum Dots for Multiphoton Fluorescence Imaging In Vivo" Science, America Association for the Advancement of Science, vol. 200, no. 5624, 1 January 2003, discloses water-soluble CdSe-ZnS quantum dots for multiphoton imaging.

### SUMMARY OF THE INVENTION

The present invention relates to a method of preparing a heteroatom doped multifunctional carbon quantum dot from a precursor according to claim 1.

Optional steps of the method are set out in the dependent claims.

The advantages implemented by the present invention are that:
1) The heteroatom doped multifunctional carbon quantum dots synthetized by the present invention are obtained by using a conjugated polymer as a precursor and through a process of high temperature carbonization, by changing the structure of the conjugated polymer, carbon quantum dots, containing one or more heteroatoms selected from the group consisting of O, N, S, Si, Se, P, As, Ge, Gd, B, Sb and Te, with different functional groups (ammonium salt, carboxyl group, amino group, aldehyde group, mercapto group, etc.) on the surface, and easy to be modified, can be obtained;
2) The heteroatom multifunctional carbon quantum dots synthetized by the present invention, having a broad absorption spectrum (300-850 nm) and adjustable light emission (350-1000 nm), can be used for the in vivo and in vitro imaging and labeling.
3) The heteroatom multifunctional carbon quantum dots prepared by the present invention have substantially no cytotoxicity in dark condition; under illumination, the quantum yields generating reactive oxygen reaches up to 40%-200%, these quantums can efficiently kill tumor cells and can be used for in vivo and in vitro photodynamic therapy/targeted therapy; and can also be used as an antibacterial agent for sterilization and killing virus at the same time.
4) The heteroatom multifunctional carbon quantum dots prepared by the present invention, under the illumination of simulated sunlight (400-800 nm), can be used for efficient photocatalytic degradation of organic pollutants and photocatalytic water-splitting for hydrogen generation.
5) The heteroatom multifunctional carbon quantum dots prepared by the present invention can be used for construction of organic polymer solar cell and quantum dot-sensitized solar cell, the efficiency of photoelectric conversion is high and can reach to more than 5%.

### DESCRIPTION OF THE DRAWINGS

Figure 1a is a graph showing the absorption spectrum and fluorescence spectrum of the synthesized green fluorescence carbon quantum dots of the present invention;
Figure 1b is a graph showing the absorption spectrum and fluorescence spectrum of the synthesized yellow fluorescence carbon quantum dots of the present invention;
Figure 1c is a graph showing the absorption spectrum and fluorescence spectrum of the synthesized red fluorescence carbon quantum dots of the present invention;
Figure 1d is a graph showing the absorption spectrum and fluorescence spectrum of the synthesized near infrared fluorescence carbon quantum dots of the present invention;
Figure 2 is a transmission electron microscopy image of the synthesized heteroatom doped carbon quantum dots of the present invention;
Figure 3 is a graph showing the effect of heteroatom doped carbon quantum dots of the present invention in the photocatalytic degradation of organic pollutants.
Figure 4 is a graph showing the effect of the heteroatom doped carbon quantum dots of the present invention on the photocatalytic water-splitting for hydrogen generation.
Figure 5 is a schematic diagram of the organic polymer solar cell constructed by using the heteroatom doped carbon quantum dots of the present invention as a new type of electron acceptor/donor material.
Figure 6 is a schematic diagram of the heteroatom doped carbon quantum dots of the present invention used for dye-sensitized solar cell.
Figure 7 is a graph showing the effect of the heteroatom doped carbon quantum dots of the present invention used for fluorescence imaging and labeling as well as photodynamic therapy; a) in vitro imaging, b) the effect of in vitro photodynamic therapy, c) in vivo imaging, and d) the effect of in vivo photodynamic therapy.
Figure 8 is a graph showing the effect of the heteroatom doped carbon quantum dots of the present invention used for antimicrobial material.

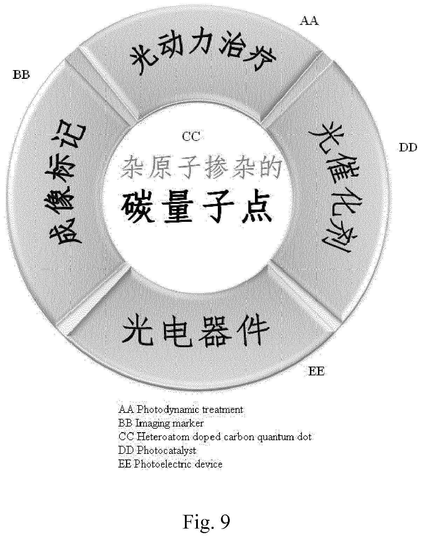

Figure 9 is a schematic diagram of the application of the heteroatom doped carbon quantum dots of the present invention.

### DETAILED DESCRIPTION OF THE INVENTION

### Example 1

A method for preparing N, P two-atom doped water-soluble carbon quantum dots, comprising the steps of:
adding 10 mg solid powder of polymer PPV1 into a beaker, adding 40 mL of aqueous solution of hydrochloric acid with a concentration of 0.5M, and mixing uniformly; transferring the mixed reaction solution into hydrothermal reaction kettle, keeping the reaction temperature at 250°C for 12 hours, and after cooling, separating and purifying to obtain N, P two-atom doped water-soluble carbon quantum dots.

An application of the above N, P two-atom doped water-soluble carbon quantum dots as a new type of photocatalyst in the degradation of organic pollutants in the environment: 100 mg of N, P two-atom doped water-soluble carbon quantum dots were diffused into 100 mL of aqueous solution of rhodamine B with a concentration of 10⁻⁵ M, then the mixed solution was transferred into a sealable quartz vessel with a condensation device, stirred for 2 hours; the mixed solution was irradiated with 450W xenon lamp of 400-800 nm wavelength at light energy of 300 mW/cm²; 2 mL of solution was taken out at intervals of 2 minutes to measure the absorbance of rhodamine B at 553 nm by UV-Vis spectrometer.

An application of the above N and P doped water-soluble carbon quantum dots as a new type of photocatalyst in the photocatalytic water-splitting for hydrogen generation: 50mg of N, P two-atom doped water-soluble carbon quantum dots were diffused into 100 mL of aqueous solution containing 10 wt% sodium ethylenediaminetetraacetate, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 500 mW/cm² for 180 minutes, and the generated hydrogen was on-line analyzed by gas chromatography.

An application of N, P two-atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1:25, then was spin-coated onto the transparent glass of indium tin oxide (ITO), with a thickness about 30 nm to form a hole transport auxiliary layer. Poly-3-hexylthiophene (P3HT) and N, P two-atom doped carbon quantum dots were dissolved in the chlorobenzene solution by weight ratio of 10: 1, and was spin-coated in 2000 rpm onto the hole transport auxiliary layer to form an active layer of 70-90 nm thickness; finally an Al electrode was evaporated with a vacuum evaporation deposition machine, with annealing at 140 °C for 10 minutes to obtain organic polymer solar cell constructed by N, P two-atom doped carbon quantum dots as a new type of electron acceptor material. The volt-ampere (I-V) characteristics of the solar cell both under illumination and in dark condition were measured.

An application of the above N, P two-atom doped water-soluble carbon quantum dots as a new type of photosensitizer in quantum dot- sensitized solar cell: titanium dioxide nanoparticles and polyethylene glycol 20000 were mixed with water by weight ratio of 25:10:65 to obtain a homogeneous white viscous slurry, and the slurry was spin-coated onto the clean surface of the FTO conductive glass to form a titanium dioxide film. The titanium dioxide film on the FTO conductive glass was heated up to 500°C, keeping the temperature for 120 minutes, to remove the organics in the film. The conductive glass electrode sintered at 500°C was cooled to 80° C, and immersed into the N, P two-atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the solar cell both under illumination and in dark condition were measured.

An application of N, P two-atom doped water-soluble carbon quantum dots as a new type of photosensitizer in the in vitro imaging and photodynamic therapy: the model for in vitro photodynamic therapy was melanoma cells. In dark condition, the melanoma cells and N, P two-atom doped water-soluble carbon quantum dots with a concentration of 20 µg/mL were incubated in cell culture solution for 24 hours. After washing twice with PBS buffered solution, the imaging and labeling effect of cells was observed under a confocal microscope. Next, after irradiation with visible light of 400-800 nm wavelength at light intensity of 100 mW/cm² for 20 minutes, these cells were continued to be incubated in cell culture incubator for 24 hours. The survival rate of melanoma cells was detected by microplate reader.

An application of N, P two-atom doped water-soluble carbon quantum dots as a new type of photosensitizer in the in vivo imaging and labeling as well as photodynamic therapy: the models for in vivo photodynamic therapy were nude mice subcutaneously inoculated with melanoma cancer cells. When the melanoma cancer tumors grew up to 30-35 mm³, 50 µL of N, P two-atom doped water-soluble carbon quantum dots of 2 mg/mL were injected into the tumors by subcutaneous injection, 2 hours later, irradiating with visible light of 400-800 nm wavelength at intensity of 100 mW/cm² for 15 minutes, once daily for two days. The in vivo imaging and labeling effect was observed by in vivo imaging system, the photographs of the nude mice and tumors after the treatment of photodynamic therapy were collected by a digital camera, and the tumour sizes were measured with vernier caliper. Two groups of comparative tests were used: one group of mice were injected with physiological saline only to let the tumors grow naturally; the other group of mice were injected with N, P two-atom doped water-soluble carbon quantum dots only, without illumination. Each group had 10 nude mice models.

Application of the above N, P two-atom doped water-soluble carbon quantum dots as a new type of photosensitizer in anti-microbial material: 200 µL of escherichia coli phosphate buffered solution of 2 × 10⁵ cfu/mL was added to sterile 24-well plates, then 10 µL of N, P two-atom doped carbon quantum dots solution of 0.5 mg/mL was added. The mixed solution was shaken and cultured for 0.5 hours in dark condition. After irradiation for 10 minutes with simulated sunlight or laser of 400-800 nm wavelength at intensity of 100 mW/cm², the mixed solution in 24-well plates was transferred to an agar plate with culture medium, and the survival rate of escherichia coli was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination.

The structural formula of polymer PPV1 was as follows:

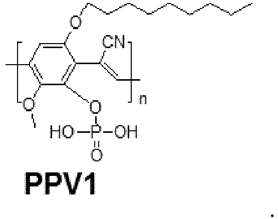

### Example 2

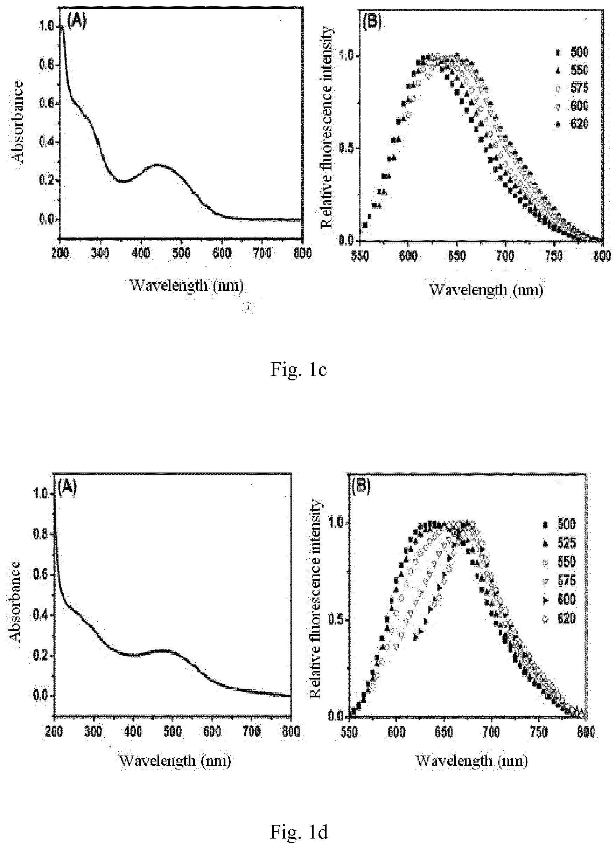

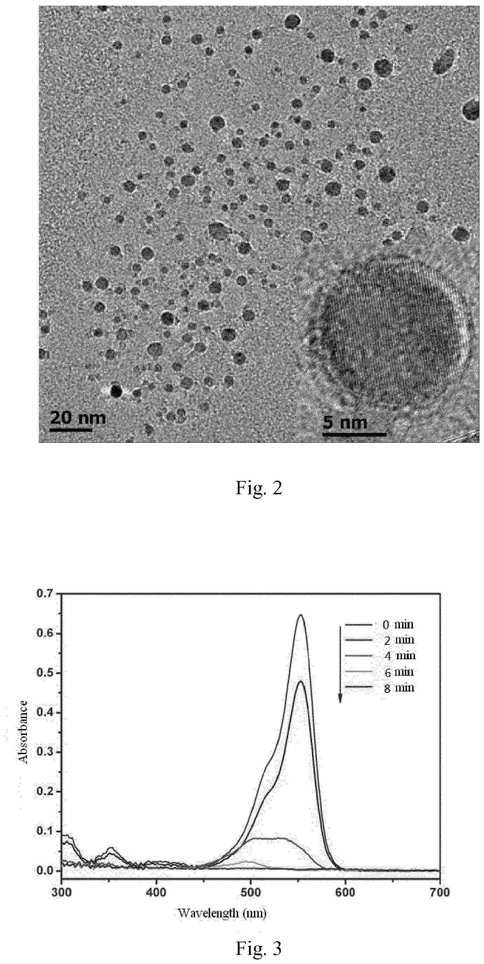

A method for preparing S, N two-atom doped water-soluble carbon quantum dots, comprising the steps of:
adding 10 mg solid powder of polymer PT1 into a beaker, adding 40 mL of aqueous solution of sulfuric acid with a concentration of 5M, and mixing uniformly; transferring the mixed reaction solution into microwave reactor, keeping the reaction temperature at 150°C for 12 hours, after cooling, separating and purifying to obtain S, N two-atom doped water-soluble carbon quantum dots. (Absorption spectrum and fluorescence spectrum were shown in Fig. 1c, and transmission electron microscope image of carbon quantum dots was shown in Fig. 2.)

An application of the above S, N two-atom doped water-soluble carbon quantum dots as a new type of photocatalyst in the degradation of organic pollutants in the environment: 100mg of S, N two-atom doped water-soluble carbon quantum dots was diffused into 100mL of aqueous solution of rhodamine B with a concentration of 10⁻⁵ M, and then the mixed solution was transferred into a sealable quartz vessel with a condensation device, stirred for 2 hours; the mixed solution was irradiated with 450W xenon lamp of 400-800nm wavelength at light energy of 300 mW/cm²; 2 mL of solution was taken out at intervals of 2 minutes to measure the absorbance of rhodamine B at 553 nm by UV-Vis spectrometer (shown in Fig. 3).

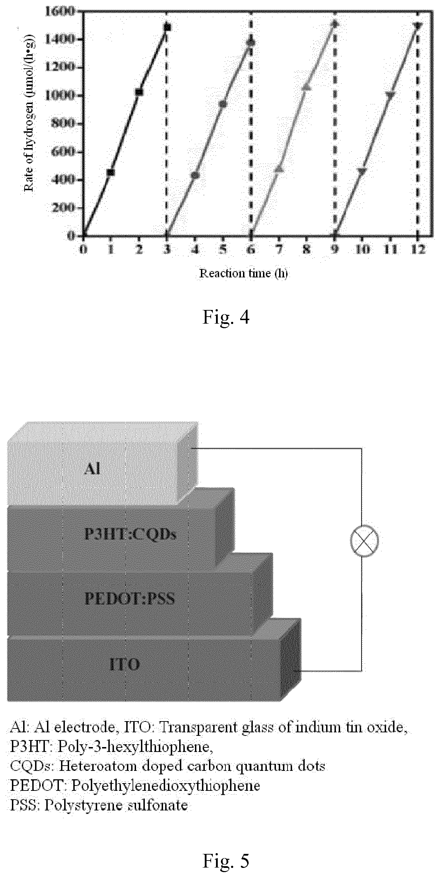

An application of the above S, N two-atom doped water-soluble carbon quantum dots as a new type of photocatalyst in the photocatalytic water-splitting for hydrogen generation: 500mg of S, N two-atom doped water-soluble carbon quantum dots were diffused into 100 mL of aqueous solution containing 10 wt% lactic acid, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 500 mW/cm² for 180 minutes, and the generated hydrogen was on-line analyzed by gas chromatography (shown in Fig. 4).

An application of the above S, N two-atom doped water-soluble carbon quantum dots as a new type of electron acceptor/donor material in the construction of organic polymer solar cell: the conductive polymer polyethylenedioxythiophene (PEDOT) was mixed with polystyrene sulfonate (PSS) by weight ratio of 1:25, then the mixture was spin-coated onto the transparent glass of indium tin oxide (ITO) to form a hole transport auxiliary layer with a thickness about 30 nm. The poly C₆₀PCBM and S, N two-atom doped carbon quantum dots were dissolved in the chlorobenzene solution by weight ratio of 10:1, the mixed solution was spin-coated onto the hole transport auxiliary layer in 2000 rpm to form an active layer with a thickness of 70-90 nm; finally an Al electrode was evaporated by a vacuum evaporation deposition machine, annealing at 140°C for 10 minutes to obtain a organic polymer solar cell constructed by S, N two-atom doped carbon quantum dots as a new type of electron acceptor material. The volt-ampere (I-V) characteristics of the cell both under illumination and in dark condition were measured.

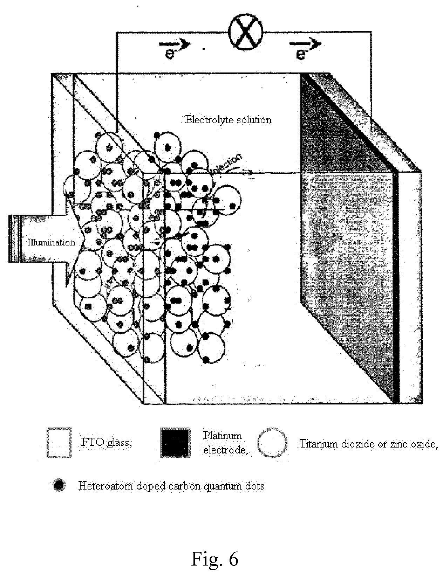

An application of the above S and N doped water-soluble carbon quantum dots as a new type of photosensitizer in the construction of quantum dot-sensitized solar cell: zinc oxide nanotubes and polyethylene glycol 20000 were mixed with water by weight ratio of 25:10:65 to obtain a homogeneous white viscous slurry, and the slurry was spin-coated onto the clean surface of the FTO conductive glass to form a titanium dioxide film. The titanium dioxide film on the FTO conductive glass was heated up to 500°C and kept for 120 minutes, to remove the organics in the film. The conductive glass electrode sintered at 500°C was cooled to 80°C, and immersed into the S, N two-atom doped carbon quantum dots aqueous solution with a concentration of 50 mg/mL, soaked for 48 hours at room temperature and in dark condition. The electrode was taken out and assembled with a platinum electrode prepared by thermal evaporation into a cell (shown in Fig.6). Electrolyte was added dropwise to the cell to complete a whole cell. The volt-ampere (I-V) characteristics of the cell both under illumination and in dark condition were measured.

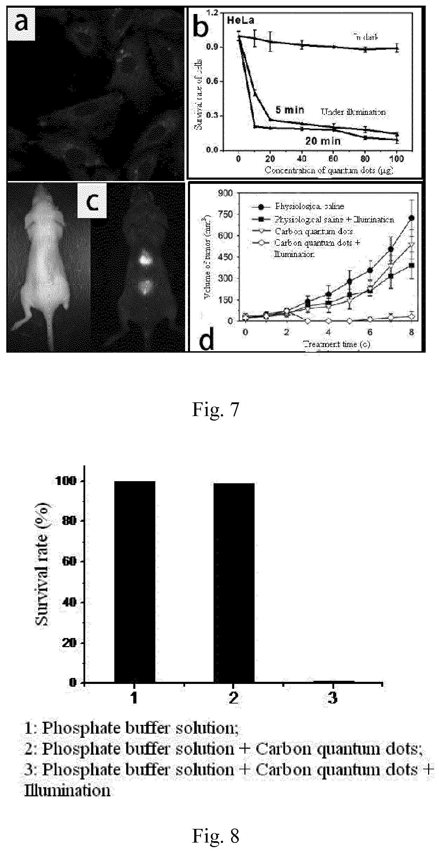

An application of the above S, N two-atom doped water-soluble carbon quantum dots as a new type of photosensitizer in the in vitro imaging and photodynamic therapy: the model for in vitro photodynamic therapy was A549 lung cancer cells. In dark condition, the A549 lung cancer cells and S, N two-atom doped water-soluble carbon quantum dots of 50 µg/mL were incubated for 24 hours in cell culture solution. After washing twice with PBS buffered solution, the imaging and labeling effect of cells was observed under a confocal microscope. Next, after being irradiated with the laser of 632nm wavelength at light intensity of 50 mW/cm² for 20 minutes, these cells were continued to be incubated for 24 hours in cell culture incubator. The survival rate of A549 lung cancer cells was detected by microplate reader (shown in Fig.7a-b).

An application of the above S, N two-atom doped water-soluble carbon quantum dots as a new type of photosensitizer in the in vivo imaging and photodynamic therapy: the model for in vivo photodynamic therapy was nude mice inoculated subcutaneously with A549 lung cancer cells. When the A549 cancer tumors grew up to 30-35 mm³, S, N two-atom doped water-soluble carbon quantum dots of 20 mg/mL were injected into the tumors by subcutaneous injection. 2 hours later, the in vivo imaging and labeling effect was collected by in vivo imaging system. Next, the tumors were irradiated with a laser of 632nm wavelength at light intensity of 150mW/cm² for 15 minutes, once daily for two days. The photographs of the nude mice and tumors after treatment of photodynamic therapy were collected by a digital camera, and the tumor sizes were measured with a vernier caliper. Three groups of comparative tests were used: the first group was injected with physiological saline only to let the tumor grow naturally; the second group was injected with S atom doped water-soluble carbon quantum dots only, without illumination; the third group was only given illumination, and each group had 10 nude mice models (shown in Fig.7c-d).

Application of the above S, N two-atom doped water-soluble carbon quantum dots as a new type of photosensitizer in anti-microbial material: 200 µL of escherichia coli phosphate buffered solution of 2×10⁵ cfu/mL was added to a sterile 24-well plate, then 10 µL of S, N two-atom doped carbon quantum dots solution with a concentration of 0.5mg/mL was added. The mixed solution was shaken and cultured for 0.5 hours in dark condition. After being irradiated with simulated sunlight or laser of 400-800nm wavelength at light intensity of 150 mW/cm² for 10 minutes, the mixed solution in 24-well plates was transferred to an agar plate with culture medium, and the survival rate of escherichia coli was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination. The structural formula of polymer PT1 was as follows:

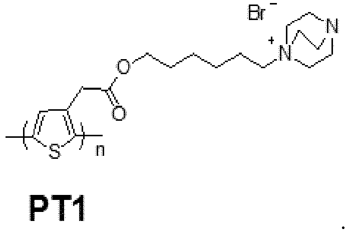

### Example 3

A method for preparing Se, N two-atom doped water-soluble carbon quantum dots, comprising the steps of:
adding 5 mg solid powder of polymer PT2 into a beaker, adding 40 mL of aqueous solution of potassium hydroxide with a concentration of 1M, and mixing uniformly; transferring the mixed reaction solution into a ultrasonic reactor, keeping the reaction temperature at 250°C for 36 hours, after cooling, separating and purifying to obtain Se, N two-atom doped water-soluble carbon quantum dots (Absorption spectrum and fluorescence spectrum were shown in Fig. 1d).

An application of Se, N two-atom doped water-soluble carbon quantum dots as a new type of photocatalyst in the degradation of organic pollutants in the environment: 100 mg of Se, N two-atom doped water-soluble carbon quantum dots was diffused into 100 mL of aqueous solution of rhodamine B of 10⁻⁵M, and then the mixed solution was transferred into a sealable quartz vessel with a condensation device, stirring for 2 hours; the mixed solution was irradiated with 450W xenon lamp of 400-800 nm wavelength at light energy of 500 mW/cm²; 2 mL of solution was taken out at intervals of 2 minutes to measure the absorbance of rhodamine B at 553 nm by UV-Vis spectrometer.

An application of Se, N two-atom doped water-soluble carbon quantum dots as a new type of photocatalyst in the photocatalytic water-splitting for hydrogen generation: 1000 mg of Se, N two-atom doped water-soluble carbon quantum dots were diffused into 100 mL of aqueous solution containing 10 wt% triethanolamine, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 800 mW/cm² for 180 minutes, and the generated hydrogen was on-line analyzed by gas chromatography.

An application of Se, N two-atom doped water-soluble carbon quantum dots as a new type of electron acceptor/donor material in the construction of organic polymer solar cell: the conductive polymer polyethylenedioxythiophene (PEDOT) was mixed with polystyrene sulfonate (PSS) by weight ratio of 1: 25, was spin-coated a hole transport auxiliary layer with a thickness about 30 nm on a transparent glass of indium tin oxide (ITO). Poly-3-hexylthiophene (P3HT) and Se, N two-atom doped carbon quantum dots were dissolved in the chlorobenzene solution by weight ratio of 10: 1, and was spin-coated in 2000 rpm onto the hole transport auxiliary layer to form an active layer with a thickness of 70-90 nm; finally an Al electrode was evaporated with a vacuum evaporation deposition machine, with annealing at 140 °C for 10 minutes, to obtain an organic polymer solar cell constructed by the Se, N two-atom doped carbon quantum dots as a new type of electron acceptor material. The volt-ampere (I-V) characteristics of the cell both under illumination and in dark condition were measured (shown in Fig. 5).

An application of the above Se, N two-atom doped water-soluble carbon quantum dots as a new type of photosensitizer in construction of quantum dot- sensitized solar cell: titanium dioxide nanotubes and polyethylene glycol 20000 were mixed with water by weight ratio of 25: 10: 65 to obtain a homogeneous white viscous slurry, and to slurry was spin-coated onto the clean surface of the FTO conductive glass to form a titanium dioxide film. The titanium dioxide film on the FTO conductive glass was heated up to 500°C, keeping the temperature for 120 minutes, to remove the organics in the film. The conductive glass electrode sintered at 500°C was cooled to 80 °C, and immersed into the Se, N two-atom doped carbon quantum dots solution with a concentration of 50 mg/mL, soaking for 48 hours at room temperature and in dark condition. The electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of Se, N two-atom doped water-soluble carbon quantum dots as a new type of photosensitizer in the in vitro targeted imaging and labeling and targeted photodynamic therapy: the models were prostate normal cells and LNCaP prostate cancer cells. The surface of Se, N two-atom doped water-soluble carbon quantum dots was modified with A10 2'-fluoropyrimidine RNA aptamers which were capable of specific recognition of prostate cancer cells. In dark condition, the prostate normal cells and LNCaP prostate cancer cells were respectively incubated in the cell culture solution with the modified water-soluble carbon quantum dots of 20 µg/mL for 6 hours. After washing twice with PBS buffered solution, the imaging and labeling data of two kinds of cells were respectively collected by a confocal microscope. Next, these cells were irradiated with visible light of 400-800 nm wavelength at light intensity of 50 mW/cm² for 20 minutes. These cells were respectively continued to be incubated for 24 hours in cell culture incubator. The survival rates of the prostate normal cells and LNCaP prostate cancer cells were detected by microplate reader.

An application of Se, N two-atom doped water-soluble carbon quantum dots used as a new type photosensitizer in the in vivo targeted imaging and labeling and targeted photodynamic therapy: the models were nude mice inoculated subcutaneously with LNCaP prostate cancer cells. When LNCaP prostate cancer tumors grew up to 30-35mm³, 200 µL of Se, N two-atom doped water-soluble carbon quantum dots with a concentration of 10 mg/mL, the surface of which was modified with A10 2'-fluoropyrimidine RNA aptamers, was injected into the mice by intravenous injection. 3 hours later, the in vivo imaging and labeling effect was collected by in vivo imaging system. Next, the tumors were irradiated with a laser of 632 nm wavelength at light intensity of 100 mW/cm² for 15 minutes, once daily for two days. The photographs of the nude mice and tumors after treatment of photodynamic therapy were collected by digital camera, and the size of tumors was measured by vernier caliper. Two groups of comparative tests were used: one group was injected physiological saline only to let the tumor grow naturally; the other group was injected the modified Se, N two-atom doped water-soluble carbon quantum dots only, without illumination. Each group had 10 nude mice models.

Application of the above Se, N two-atom doped water-soluble carbon quantum dots as a new type of photosensitizer in anti-microbial material: 200 µL of staphylococcus aureus phosphate buffered solution of 2× 10⁵ cfu/mL was added to sterile 24-well plates, then 10 µL of Se, N two-atom doped carbon quantum dots solution with a concentration of 0.5 mg/mL was added. The mixed solution was shaken and cultured for 0.5 hours in dark condition. After being irradiated with simulated sunlight of 400-800 nm wavelength at light intensity of 100 mW/cm² for 10 minutes, the mixed solution in 24-well plates was transferred to an agar plate with medium, and the survival rate of staphylococcus aureus was calculated by colony counting method. In addition, two groups of comparative tests were used: one group was that the phosphate buffered solution was mixed with bacterium suspension, without illumination; the other group was that phosphate buffered solution and aqueous solution of carbon quantum dots were mixed with bacterium suspension, without illumination. The structural formula of polymer PT2 was as follows:

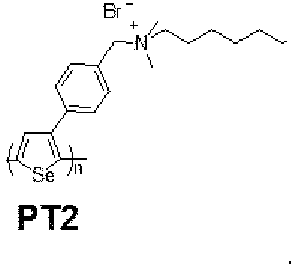

### Example 4

A method for preparing S, N, P three-atom doped water-soluble carbon quantum dots, comprising the steps of:
adding mixed solid powders consisted of 10 mg of polymer PT5 and 10 mg of polymer PPP1 into a beaker, adding 40 mL of aqueous solution of phosphoric acid with a concentration of 0.5M, and mixing uniformly; transferring the mixed reaction solution into a hydrothermal reaction kettle, keeping the reaction temperature at 200 °C for 12 hours, and after cooling, separating and purifying to obtain S, N and P three-atom doped water-soluble carbon quantum dots.

An application of the above S, N and P three-atom doped water-soluble carbon quantum dots as a new type of photocatalyst in the degradation of organic pollutants in the environment: 100 mg of S, N and P three-atom doped water-soluble carbon quantum dots were diffused into 100 mL of aqueous solution of rhodamine B with a concentration of 10⁻⁵M, then the mixed solution was transferred into a sealable quartz vessel with a condensation device, stirred for 2 hours; the mixed solution was irradiated with 450W xenon lamp of 400-800 nm wavelength at light energy of 1000 mW/cm²; 2 mL of solution was taken out at intervals of 2 minutes to measure the absorbance of rhodamine B at 553 nm by UV-Vis spectrometer.

An application of the above S, N and P three-atom doped water-soluble carbon quantum dots as a new type of photocatalyst in the photocatalytic water-splitting for hydrogen generation: 50 mg of S, N and P three-atom doped water-soluble carbon quantum dots were diffused into 100 mL of aqueous solution containing 10 wt% methanol, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 1500 mW/cm² for 180 minutes, and the generated hydrogen was on-line analyzed by gas chromatography.

An application of the above S, N and P three-atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated the transparent glass of indium tin oxide (ITO), with a thickness about 30 nm to form a hole transport auxiliary layer. Poly-3-hexylthiophene (P3HT) and S, N, P doped carbon quantum dots were dissolved in the chlorobenzene solution by weight ratio of 10: 1, and was spin-coated in 2000 rpm onto the hole transport auxiliary layer to form an active layer of 70-90 nm thickness;finally Al electrode was evaporated with a vacuum evaporation deposition machine, with annealing at 140 °C for 10 minutes to obtain organic polymer solar cell constructed by S, N and P atoms doped carbon quantum dots as a new type of electron acceptor material. The volt-ampere (I-V) characteristics of the cell both under illumination and in dark condition were measured.

An application of the above S, N and P three-atom doped water-soluble carbon quantum dots as a new type of photosensitizer in quantum dot-sensitized solar cell: zinc oxide nanotubes, and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on FTO conductive glass was heated up to 500°C, keeping the temperature for 120 minutes, to remove the organics in the film. The conductive glass electrode sintered at 500°C was cooled to 80 °C, and immersed into the S, N and P three-atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of the above S, N and P three-atom doped water-soluble carbon quantum dots as a new type of photosensitizer in the in vitro imaging and photodynamic therapy: the model was XPA1 pancreatic cancer cells. In dark condition, the XPA1 pancreatic cells and the S, N and P three-atom doped water-soluble carbon quantum dots with a concentration of 20 µg/mL were incubated respectively in cell culture solution for 10 hours. After washing twice with PBS buffered solution, the imaging and labeling effect of cells was observed under a confocal microscope. Next, the cancer cells were irradiated with visible light of 400-800 nm wavelength at light intensity of 50 mW/cm² for 20 minutes. The cancer cells were continued to be incubated in cell culture incubator for 24 hours. The survival rate of XPA1 pancreatic cancer cells was detected by microplate reader.

An application of the above S, N and P three-atom doped water-soluble carbon quantum dots as a new type of photosensitizer in the in vivo imaging and labeling and photodynamic therapy: the models were a nude mice subcutaneously inoculated with XPA1 pancreatic cancer cells. When the XPA1 pancreatic cancer tumors grew up to 30-35 mm³, 200 µL of S, N and P three-atom doped water-soluble carbon quantum dots with a concentration of 20 mg/mL were injected into the tumors by subcutaneous injection. One hour later, the in vivo imaging and labeling effect was observed by in vivo imaging system. Next, the tumors were irradiated with visible light of 400-800 nm wavelength at light intensity of 120 mW/cm² for 15 minutes, once daily for two days. The photographs of the nude mice and tumors after the treatment of photodynamic therapy were collected by a digital camera, and the tumor sizes were measured with vernier caliper. Two groups of comparative tests were used: one group of mice were injected with physiological saline only to let the tumors grow naturally; the other group of mice were injected with S, N and P three-atom doped water-soluble red fluorescence carbon quantum dots only, without illumination. Each group had 10 nude mice models.

Application of the above S, N and P three-atom doped water-soluble carbon quantum dots as a new type of photosensitizer in anti-microbial material: 200 µL of escherichia coli phosphate buffered solution of 2× 10⁵ cfu/mL was added to sterile 24-well plates, then 10 µL of S, N and P three-atom doped carbon quantum dots solution with a concentration of 1.0 mg/mL was added. The mixed solution was shaken and cultured for 0.5 hours in dark condition. After being irradiated for 10 minutes with simulated sunlight of 400-800 nm wavelength at light intensity of 150 mW/cm², the mixed solution in 24-well plates was transferred to an agar plate with culture medium, and the survival rate of escherichia coli was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination. The structural formulas of polymer PPP1 and PT5 were as follows:

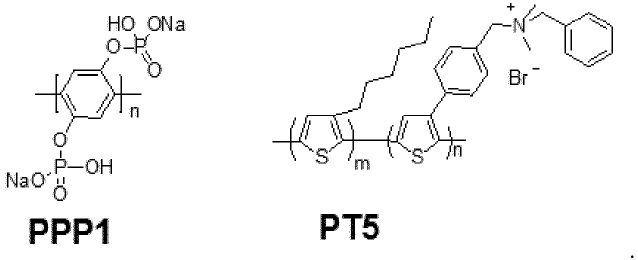

### Example 5

A method for preparing Se, N two-atom doped water-soluble carbon quantum dots, comprising the steps of:
adding 5 mg solid powder of polymer PT8 into a beaker, adding 40 mL of aqueous solution of potassium hydroxide with a concentration of 1 M, and mixing uniformly; transferring the mixed reaction solution into hydrothermal reaction kettle, keeping the reaction temperature at 250 °C for 36 hours, after cooling, separating and purifying to obtain Se, N two-atom doped water-soluble carbon quantum dots.

An application of the above Se, N two-atom doped water-soluble carbon quantum dots as a new type of photocatalyst in the degradation of organic pollutants in the environment: 100 mg of Se, N two-atom doped water-soluble carbon quantum dots was diffused into 100 mL of aqueous solution of rhodamine B of 10⁻⁵M, and then the mixed solution was transferred into a sealable quartz vessel with a condensation device, stirred for 2 hours; the mixed solution was irradiated with 450W xenon lamp of 400-800 nm wavelength at light energy of 800 mW/cm²; 2 mL of solution was taken out at intervals of 2 minutes to measure the absorbance of rhodamine B at 553 nm by UV-Vis spectrometer.

An application of the above Se, N two-atom doped water-soluble carbon quantum dots as a new type of photocatalyst in the photocatalytic water-splitting for hydrogen generation: 1000 mg of Se, N two-atom doped water-soluble carbon quantum dots were diffused into 100 mL of aqueous solution containing 10 wt% triethanolamine, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 1200 mW/cm² for 180 minutes, and the generated hydrogen was on-line analyzed by gas chromatography.

An application of the above Se, N two-atom doped water-soluble carbon quantum dots as a new type of electron acceptor/donor material in the construction of organic polymer solar cell: the conductive polymer polyethylenedioxythiophene (PEDOT) was mixed with polystyrene sulfonate (PSS) by weight ratio of 1: 25, then the mixture was spin-coated onto the transparent glass of indium tin oxide (ITO)to form a hole transport auxiliary layer with a thickness of about 30 nm. C₇₀PCBM and Se, N two-atom doped carbon quantum dots were dissolved in the chlorobenzene solution by weight ratio of 10: 1, the mixed solution was spin-coated onto the hole transport auxiliary layer in 2000 rpm to form an active layer with a thickness of 70-90 nm; finally an Al electrode was evaporated by a vacuum evaporation deposition machine, annealing at 140 °C for 10 minutes to obtain a organic polymer solar cell constructed by Se, N two-atom doped carbon quantum dots as a new type of electron acceptor material. The volt-ampere (I-V) characteristics of the cell both under illumination and in dark condition were measured.

An application of the above Se, N two-atom doped water-soluble carbon quantum dots as a new type of photosensitizer in the construction of quantum dot-sensitized solar cell: titanium dioxide nanotubes and polyethylene glycol 20000 were mixed with water by weight ratio of 25: 10: 65 to obtain a homogeneous white viscous slurry, and the slurry was spin-coated onto the clean surface of the FTO conductive glass to form a film. The titanium dioxide film on the FTO conductive glass was heated up to 500°C and kept for 120 minutes, to remove the organics in the film. The conductive glass electrode sintered at 500 °C was cooled to 80 °C, and immersed into the Se, N two-atom doped carbon quantum dots aqueous solution with a concentration of 50 mg/mL, soaked for 60 hours at room temperature and in dark condition. The electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of the above Se, N two-atom doped water-soluble carbon quantum dots as a new type of photosensitizer in the in vitro targeted imaging and labeling and targeted photodynamic therapy: the models were PC3 prostate cancer cells and prostate normal cells. In dark condition, the PC3 prostate cancer cells and prostate normal cells were respectively incubated with the Se, N two-atom water-soluble carbon quantum dots with a concentration of 20 µg/mL, the surface of which was modified with folic acid, for 6 hours in cell culture solution. After washing twice with PBS buffered solution, the imaging and labeling data of two kinds of cells were respectively collected by a confocal microscope. Next, these cells were irradiated for 20 minutes with visible light of 400-800 nm wavelength at light intensity of 50 mW/cm². These cells were continued respectively to be incubated for 24 hours in cell culture incubator. The survival rates of the PC3 prostate cancer cells and prostate normal cells and were detected by microplate reader.

An application of the above Se, N two-atom doped water-soluble carbon quantum dots as a new type photosensitizer in the in vivo targeted imaging and labeling and targeted photodynamic therapy: the models were nude mice inoculated subcutaneously with PC3 prostate cancer cells. When PC3 prostate cancer tumors grew up to 30-35mm³, 200 µL of Se, N two-atom doped water-soluble carbon quantum dots with a concentration of 10 mg/mL, the surface of which was modified with folic acid, was injected into the mice by intravenous injection. 3 hours later, the in vivo imaging and labeling effect was collected by in vivo imaging system. Next, the tumors were irradiated with laser of 632 nm wavelength at light intensity of 100 mW/cm² for 15 minutes, once daily for two days. The photographs of the nude mice and tumors after treatment of photodynamic therapy were collected by a digital camera, and the tumor sizes were measured with a vernier caliper. Two groups of comparative tests were used: one group was injected physiological saline only to let the tumor grow naturally; the other group was injected the modified Se, N two-atom doped water-soluble carbon quantum dots only, without illumination. Each group had 10 nude mice models.

Application of the above Se, N two-atom doped water-soluble carbon quantum dots as a new type of photosensitizer in anti-microbial material: 200 µL of staphylococcus aureus phosphate buffered solution of 2×10⁵ cfu/mL was added to sterile 24-well plates, then 10 µL of Se, N two-atom doped carbon quantum dots solution with a concentration of 0.5 mg/mL was added. The mixed solution was shaken and cultured for 0.5 hours in dark condition. After being irradiated for 10 minutes with simulated sunlight of 400-800 nm wavelength at light intensity of 100 mW/cm², the mixed solution in 24-well plates was transferred to an agar plate with culture medium, and the survival rate of staphylococcus aureus was calculated by colony counting method. In addition, two groups of comparative tests were used: one group was that the phosphate buffered solution was mixed with bacterium suspension, without illumination; the other group was that phosphate buffered solution and aqueous solution of carbon quantum dots were mixed with bacterium suspension, without illumination. The structural formula of polymer PT8 was as follows:

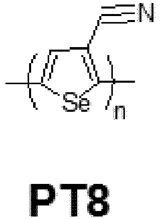

### Example 6

A method for preparing N atom doped water-soluble carbon quantum dots, comprising the steps of:
adding 30 mg solid powder of polymer PT6 into a beaker, adding 40 mL of aqueous solution of potassium hydroxide with a concentration of 0.5 M, and mixing uniformly; transferring the mixed reaction solution into hydrothermal reaction kettle, keeping the reaction temperature at 210 °C for 10 hours, after cooling, separating and purifying to obtain N atom doped carbon quantum dots.

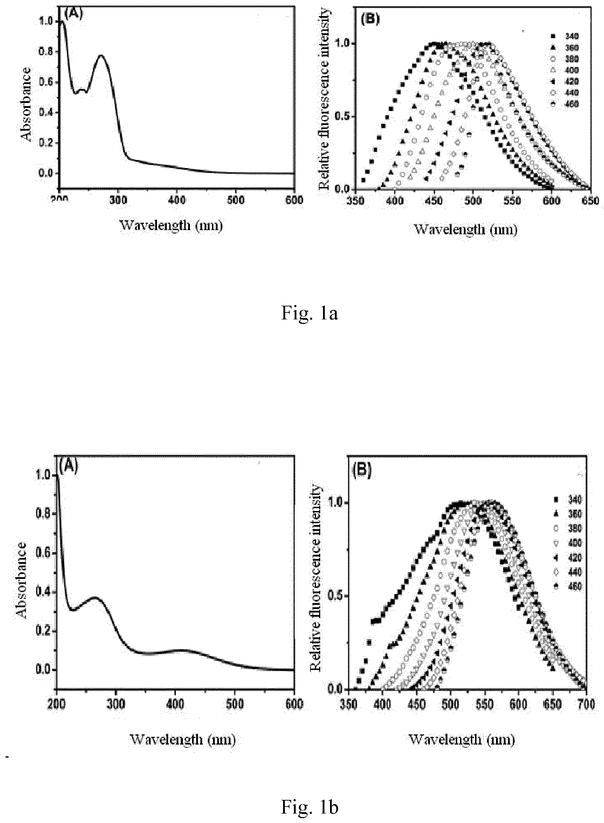

(Absorption spectrum and fluorescence spectrum were shown in Fig. 1a)

An application of N atom doped water-soluble carbon quantum dots as a new type of photocatalyst in the degradation of organic pollutants in the environment: 100 mg of N atom doped water-soluble carbon quantum dots was diffused into 100 mL of aqueous solution of rhodamine B of 10⁻⁵M, and then the mixed solution was transferred into a sealable quartz vessel with a condensation device, stirred for 2 hours; the mixed solution was irradiated with 450W xenon lamp of 400-800 nm wavelength at light energy of 300 mW/cm²; 2 mL of solution was taken out at intervals of 2 minutes to measure the absorbance of rhodamine B at 553 nm by UV-Vis spectrometer.

An application of N atom doped water-soluble carbon quantum dots as a new type of photocatalyst in the photocatalytic water-splitting for hydrogen generation: 500 mg of N atom doped water-soluble carbon quantum dots were diffused into 100 mL of aqueous solution containing 10 wt% triethanolamine, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 500 mW/cm² for 180 minutes, and the generated hydrogen was on-line analyzed by gas chromatography.

An application of N atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated onto the transparent glass of indium tin oxide (ITO) with a thickness about 30 nm to form a hole transport auxiliary layer. Poly-3-hexylthiophene (P3HT) and N atom doped carbon quantum dots were dissolved in the chlorobenzene solution by weight ratio of 10: 1, and spin-coated in 2000 rpm onto the hole transport auxiliary layer to form an active layer of 70-90 nm; finally Al electrode was evaporated with a vacuum evaporation deposition machine, with annealing at 140 °C for 10 minutes to obtain organic polymer solar cell constructed by N atom doped carbon quantum dots as a new type of electron acceptor material. The volt-ampere (I-V) characteristics of the solar cell both under illumination and in dark condition were measured.

An application of the above N atom doped water-soluble carbon quantum dots as a new type of photosensitizer in quantum dot-sensitized solar cell: titanium dioxide nanotubes and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on FTO conductive glass was heated up to 500°C, keeping the temperature for 120 minutes, to remove the organics in the film. The conductive glass electrode sintered at 500°C was cooled to 80 °C, and immersed into the N atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of N atom doped water-soluble carbon quantum dots as a new type of photosensitizer in the in vitro imaging and photodynamic therapy: the model for in vitro photodynamic therapy was SCC25 tongue cancer cells. In dark condition, the SCC25 tongue cancer cells and N atom doped water-soluble carbon quantum dots with a concentration of 20 µg/mL were incubated for 24 hours in cell culture solution. After washing twice with PBS buffered solution, the imaging and labeling effect of cells was observed under a confocal microscope. Next, after irradiation with visible light of 400-800 nm wavelength at light intensity of 50 mW/cm² for 18 minutes, these cells were continued to be incubated for 24 hours in cell culture incubator. The survival rate of SCC25 tongue cancer cells was detected by microplate reader.

An application of N atom doped water-soluble carbon quantum dots as a new type of photosensitizer in the in vivo imaging and labeling and photodynamic therapy: the model for in vivo photodynamic therapy was nude mice subcutaneously inoculated with SCC25 tongue cancer cells. When the SCC25 tongue cancer tumors grew up to 30-35 mm³, 200 µL of N atom doped water-soluble carbon quantum dots with a concentration of 2 mg/mL were injected into the tumors by subcutaneous injection. 2 hours later, the in vivo imaging and labeling effect was observed by in vivo imaging system. Next, these tumors were irradiated for 15 minutes with visible light of 400-800 nm wavelength at light intensity of 100 mW/cm², once daily for two days. The photographs of the nude mice and tumors after the treatment of photodynamic therapy were collected by a digital camera, and the tumor sizes were measured with vernier caliper. Two groups of comparative tests were used: one group of mice were injected with physiological saline only to let the tumors grow naturally; the other group of mice were injected with N atom doped water-soluble red fluorescence carbon quantum dots only, without illumination. Each group had 10 nude mice models.

Application of the above N atom doped water-soluble carbon quantum dots as a new type of photosensitizer in anti-microbial material: 200 µL of bacteriophage phosphate buffered solution with a concentration of 2×10⁵ cfu/mL was added to sterile 24-well plates, then 10 µL of N atom doped carbon quantum dots solution with a concentration of 2.0 mg/mL was added. The mixed solution was shaken and cultured for 0.5 hours in dark condition. After being irradiated for 10 minutes with simulated sunlight of 400-800 nm wavelength at light intensity of 50 mW/cm², the mixed solution in 24-well plates was transferred to an agar plate with medium, and the survival rate of bacteriophage was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination (shown in Fig. 8).

The structural formula of polymer PT6 was as follows:

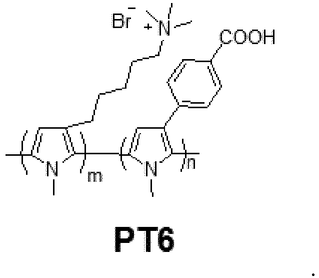

### Example 7

A method for preparing N, S two-atom doped water-soluble carbon quantum dots, comprising the steps of:
adding 10 mg solid powder of polymer PF1 into a beaker, adding 40 mL of aqueous solution of sodium hydroxide with a concentration of 5 M, and mixing uniformly; transferring the mixed reaction solution into microwave reactor, keeping the reaction temperature at 250 °C for 48 hours, after cooling, separating and purifying to obtain the N, S two-atom doped water-soluble carbon quantum dots.

An application of the above S, N two-atom doped water-soluble carbon quantum dots as a new type of photocatalyst in the degradation of organic pollutants in the environment: 100 mg of S, N two-atom doped water-soluble carbon quantum dots was diffused into 100 mL of aqueous solution of rhodamine B of 10⁻⁵M, and then the mixed solution was transferred into a sealable quartz vessel with a condensation device, stirred for 2 hours; the mixed solution was irradiated with 450W xenon lamp of 400-800 nm wavelength at light energy of 400 mW/cm²; 2 mL of solution was taken out at intervals of 2 minutes to measure the absorbance of rhodamine B at 553 nm by UV-Vis spectrometer.

An application of the above S, N two-atom doped water-soluble carbon quantum dots as a new type of photocatalyst in the photocatalytic water-splitting for hydrogen generation: 500 mg of S, N two-atom doped water-soluble carbon quantum dots were diffused into 100 mL of aqueous solution containing 10 wt% lactic acid, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 1500 mW/cm² for minutes, and the generated hydrogen was on-line analyzed by gas chromatography.

An application of the above S, N two-atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated onto the transparent glass of indium tin oxide (ITO) with a thickness about 30 nm to form a hole transport auxiliary layer. Poly-3-hexylthiophene (P3HT) and S, N two-atom doped carbon quantum dots were dissolved in the chlorobenzene solution by weight ratio of 10:1, and was spin-coated in 2000 rpm onto the hole transport auxiliary layer to form an active layer of 70-90 nm thickness; finally Al electrode was evaporated with a vacuum evaporation deposition machine, with annealing at 140 °C for 10 minutesto obtain organic polymer solar cell constructed by S, N two-atom doped carbon quantum dots as a new type of electron acceptor material. The volt-ampere (I-V) characteristics of the cell both under illumination and in dark condition were measured.

An application of the above S, N two-atom doped water-soluble carbon quantum dots as a new type of photosensitizer in quantum dot- sensitized solar cell: zinc oxide nanotubes and polyethylene glycol 20000 were mixed with water by weight ratio of 25:10:65 to obtain a homogeneous white viscous slurry, and the slurry was spin-coated onto the clean surface of the FTO conductive glass to form a titanium dioxide film. The titanium dioxide film on FTO conductive glass was heated up to 500 °C, keeping the temperature for 120 minutes, to remove the organics in the film. The conductive glass electrode sintered at 500°C was cooled to 80 °C, and immersed into the S, N two-atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of S, N two-atom doped water-soluble carbon quantum dots as a new type of photosensitizer in the in vitro imaging and photodynamic therapy: the models for in vitro photodynamic therapy were MCF7 breast cancer cells. In dark condition, the MCF7 breast cancer cells and S, N two-atom doped water-soluble carbon quantum dots with a concentration of 50 µg/mL were incubated in cell culture solution for 4 hours. After washing twice with PBS buffered solution, the imaging and labeling effect of cells was observed under a confocal microscope. Next, after irradiation for 20 minutes with laser of 632 nm wavelength at light intensity of 50 mW/cm², these cells were continued to be incubated in cell culture incubator for 24 hours. The survival rate of MCF7 breast cancer cells was detected by microplate reader.

An application of S, N two-atom doped water-soluble carbon quantum dots as a new type of photosensitizer in the in vivo imaging and labeling and photodynamic therapy: the models for in vivo photodynamic therapy were nude mice subcutaneously inoculated with MCF7 breast cancer cells. When the MCF7 breast cancer tumors grew up to 30-35 mm³, S, N two-atom doped water-soluble carbon quantum dots with a concentration of 6 mg/mL were injected into the tumors by subcutaneous injection. 2 hours later, the in vivo imaging and labeling effect was collected by in vivo imaging system. Next, the tumors were irradiated for 15 minutes with laser of 632 nm wavelength at light intensity of 200 mW/cm², once daily for two days. The photographs of the nude mice and tumors after the treatment of photodynamic therapy were collected by a digital camera, and the tumor sizes were measured with vernier caliper. Three groups of comparative tests were used: the first group of mice were injected with physiological saline only to let the tumor grow naturally; the second group of mice were injected with S atom doped water-soluble carbon quantum dots only, without illumination; the third group of mice were only given illumination, and each group had 10 nude mice models.

Application of the above S, N two-atom doped water-soluble carbon quantum dots as a new type of photosensitizer in anti-microbial material: 200 µL of escherichia coli phosphate buffered solution of 2×10⁵ cfu/mL was added to sterile 24-well plates, then 10 µL of S, N two-atom doped carbon quantum dot solution with a concentration of 0.5mg/mL was added. The mixed solution was shaken and cultured for 0.5 hours in dark condition. After being irradiated for 10 minutes with laser of 400-800 nm wavelength at light intensity of 200 mW/cm², the mixed solution in 24-well plates was transferred to an agar plate with culture medium, and the survival rate of escherichia coli was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination.

The structural formula of polymer PF1 was as follows:

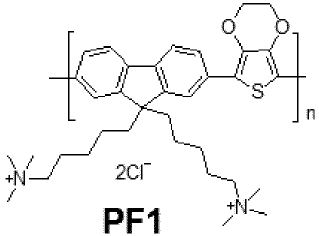

### Example 8

A method for preparing P atom doped water-soluble carbon quantum dots, comprising the steps of:
adding 10 mg solid powder of polymer PPP1 into a beaker, adding 40 mL of aqueous solution of phosphoric acid with a concentration of 0.5M, and mixing uniformly; transferring the mixed reaction solution into hydrothermal reaction kettle, keeping the reaction temperature at 500 °C for 12 hours, and after cooling, separating and purifying to obtain P atom doped water-soluble carbon quantum dots.

An application of the above P atomic water-soluble carbon quantum dots as a new type of photocatalyst in the degradation of organic pollutants in the environment: 100 mg of P atom doped water-soluble carbon quantum dots were diffused into 100 mL of aqueous solution of methyl orange with a concentration of 10⁻⁵M, then the mixed solution was transferred into a sealable quartz vessel with a condensation device, stirred for 2 hours; the mixed solution was irradiated with 450W xenon lamp of 400-800 nm wavelength at light energy of 900 mW/cm²; 2 mL of solution was taken out at intervals of 2 minutes to measure the absorbance of methyl orange at 463 nm by UV-Vis spectrometer.

An application of the above P atom doped water-soluble carbon quantum dots as a new type of photocatalyst in the photocatalytic water-splitting for hydrogen generation: 900 mg of P atom doped water-soluble carbon quantum dots were diffused into 100 mL of aqueous solution containing 10 wt% sodium sulfide, the mixed solution was transferred into a sealable quartz vessel with a condensation device, and high purity nitrogen was introduced to remove the dissolved oxygen in the water completely. Then, the mixed solution was irradiated by 450W xenon lamp of 400-800 nm wavelength at energy of 1000 mW/cm² for 180 minutes, and the generated hydrogen was on-line analyzed by gas chromatography.

An application of the above P atom doped water-soluble carbon quantum dots as a new type of electron acceptor/donor material in the construction of organic polymer solar cell: the conductive polymer polyethylenedioxythiophene (PEDOT) was mixed with polystyrene sulfonate (PSS) by weight ratio of 1: 25, then the mixture was spin-coated onto the transparent glass of indium tin oxide (ITO) to form a hole transport auxiliary layer with a thickness about 30 nm. The poly-3-hexylthiophene (P3HT) and P atom doped carbon quantum dots were dissolved in the chlorobenzene solution by weight ratio of 10: 1, the mixed solution was spin-coated onto the hole transport auxiliary layer in 2000 rpm to form an active layer with a thickness of 70-90 nm; finally an Al electrode was evaporated by a vacuum evaporation deposition machine, annealing at 140 °C for 10 minutes to obtain a organic polymer solar cell constructed by P atom doped carbon quantum dots as a new type of electron acceptor material. The volt-ampere (I-V) characteristics of the cell both under illumination and in dark condition were measured.

An application of the above P atom doped water-soluble carbon quantum dots as a new type of photosensitizer in the construction of quantum dot-sensitized solar cell: zinc oxide nanowires, and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on the FTO conductive glass was heated up to 500°C, and kept for 120 minutes, to remove the organics in the film. The conductive glass electrode sintered at 500 °C was cooled to 80 °C, and immersed into the P atom doped carbon quantum dots aqueous solution with a concentration of 50 mg/mL, soaked for 48 hours at room temperature and in dark condition. The electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of the above P atom doped water-soluble carbon quantum dots as a new type of photosensitizer in the in vitro imaging and photodynamic therapy: the model for in vitro photodynamic therapy was Hep2 laryngeal cancer cells. In dark condition, the Hep2 laryngeal cancer cells and the P atom doped water-soluble carbon quantum dots with a concentration of 200 µg/mL were incubated respectively in cell culture solution for 4 hours. After washing twice with PBS buffered solution, the imaging and labeling effect of cells was observed under a confocal microscope. Next, the cancer cells were irradiated with visible light of 400-800 nm wavelength at light intensity of 100 mW/cm² for 20 minutes. The cancer cells were continued to be incubated in cell culture incubator for 48 hours, and the survival rate of Hep2 laryngeal cancer cells was detected by microplate reader.

An application of the above P atom doped water-soluble carbon quantum dots as a new type of photosensitizer in the in vivo imaging and labeling and photodynamic therapy: the models for in vivo photodynamic therapy were nude mice subcutaneously inoculated with Hep2 laryngeal cancer cells. When the Hep2 laryngeal cancer cells tumors grew up to 30-35 mm³, 100 µL of P atom doped water-soluble carbon quantum dots with a concentration of 2 mg/mL were injected into the tumors by subcutaneous injection. 2 hours later, the in vivo imaging and labeling effect was observed by in vivo imaging system. Next, the tumors were irradiated with visible light of 400-800 nm wavelength at light intensity of 100 mW/cm² for 15 minutes, once daily for two days. The photographs of the nude mice and tumors after the treatment of photodynamic therapy were collected by a digital camera, and the tumor sizes were measured by a vernier caliper. Two groups of comparative tests were used: one group was injected with physiological saline only to let the tumors grow naturally; the other group was injected with P atom doped water-soluble carbon quantum dots only, without illumination. Each group had 10 nude mice models.

Application of the P atom doped water-soluble carbon quantum dots as a new type of photosensitizer in anti-microbial material: 200 µL of treponema pallidum phosphate buffered solution of 2×10⁵ cfu/mL was added to a sterile 24-well plates, then 10 µL of P atom doped carbon quantum dots solution with a concentration of 1.0 mg/mL was added. The mixed solution was shaken and cultured for 0.5 hours in dark condition. After being irradiated with simulated sunlight or laser of 400-800 nm wavelength at light intensity of 100 mW/cm² for 10 minutes, the mixed solution in 24-well plates was transferred to an agar plate with culture medium, and the survival rate of treponema pallidum was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination. The structural formula of polymer PPP1 was as follows:

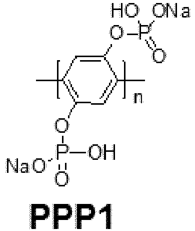

### Example 9

A method for preparing Se atom doped water-soluble carbon quantum dots, comprising the steps of:
adding 5 mg solid powder of polymer PT3 into a beaker, adding 40 mL of aqueous solution of potassium hydroxide with a concentration of 0.5 mM, and mixing uniformly; transferring the mixed reaction solution into hydrothermal reaction kettle, keeping the reaction temperature at 200 °C for 12 hours, after cooling, separating and purifying to obtain Se atom doped water-soluble carbon quantum dots. (Absorption spectrum and fluorescence spectrum were shown in Fig. 1b)

An application of the above Se atom doped water-soluble carbon quantum dots as a new type of photocatalyst in the degradation of organic pollutants in the environment: 100 mg of Se atom doped water-soluble carbon quantum dots was diffused into 100 mL of aqueous solution of rhodamine B of 10⁻⁵ M, and then the mixed solution was transferred into a sealable quartz vessel with a condensation device, stirred for 2 hours; the mixed solution was irradiated with 450W xenon lamp of 400-800 nm wavelength at light energy of 100 mW/cm²; 2 mL of solution was taken out at intervals of 2 minutes to measure the absorbance of rhodamine B at 553 nm by UV-Vis spectrometer.

An application of the above Se atom doped water-soluble carbon quantum dots as a new type of photocatalyst in the photocatalytic water-splitting for hydrogen generation: 800 mg of Se atom doped water-soluble carbon quantum dots were diffused into 100 mL of aqueous solution containing 10 wt% potassium iodide, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 500 mW/cm² for 180 minutes, and the generated hydrogen was on-line analyzed by gas chromatography.

An application of the above Se atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated onto the transparent glass of indium tin oxide (ITO) with a thickness about 30 nm to form a hole transport auxiliary layer. Poly-3-hexylthiophene (P3HT) and Se atom doped carbon quantum dots were dissolved in the chlorobenzene solution by weight ratio of 10: 1, and was spin-coated in 2000 rpm on to the hole transport auxiliary layer to form an active layer of 70-90 nm thickness ; finally an Al electrode was evaporated with a vacuum evaporation deposition machine, with annealing at 140 °C for 10 minutesto obtain organic polymer solar cell constructed by Se atom doped carbon quantum dots as a new type of electron acceptor material. The volt-ampere (I-V) characteristics of the solar cell both under illumination and in dark condition were measured.

An application of the above Se atom doped water-soluble carbon quantum dots as a new type of photosensitizer in quantum dot-sensitized solar cell: titanium dioxide nanowires and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on the FTO conductive glass was heated up to 500°C, keeping the temperature for 120 minutes, to remove the organics in the film. The conductive glass electrode sintered at 500°C was cooled to 80 °C, and immersed into the Se atom doped carbon quantum dots aqueous solution with a concentration of 50 mg/mL, soaking for 50 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of the above Se atom doped water-soluble carbon quantum dots as a new type of photosensitizer in the in vitro imaging and photodynamic therapy: the models for in vitro photodynamic therapy were KU7 bladder cancer cells. In dark condition, the KU7 bladder cancer cells and the Se atom water-soluble carbon quantum dots with a concentration of 200 µg/mL were incubated for 12 hours in cell culture solution. After washing twice with PBS buffered solution, the labeling effect of cells was observed under a confocal microscope. Next, these cells were irradiated with visible light of 400-800 nm wavelength at light intensity of 200 mW/cm² for 20 minutes. Then these cells were continued to be incubated in cell culture incubator for 48 hours. The survival rate of the KU7 bladder cancer cells was detected by microplate reader.

An application of the above Se atom doped water-soluble carbon quantum dots as a new type photosensitizer in the in vivo imaging and photodynamic therapy: the models for in vivo photodynamic therapy were nude mice subcutaneously inoculated with KU7 bladder cancer cells. When KU7 bladder cancer tumors grew up to 30-35mm³, 100 µL of Se atom doped water-soluble carbon quantum dots with a concentration of 3 mg/mL was injected into the tumors by intravenous injection. One hour later, the in vivo imaging and labeling effect was observed by in vivo imaging system. Next, the tumors were irradiated with visible light of 400-800 nm wavelength at light intensity of 100 mW/cm² for 15 minutes, once daily for two days. The photographs of the nude mice and tumors after treatment of photodynamic therapy were collected by a digital camera, and the tumor sizes were measured by vernier caliper. Two groups of comparative tests were used: one group of mice were injected with physiological saline only to let the tumor grow naturally; the other group of mice were injected with the Se atom doped water-soluble carbon quantum dots only, without illumination. Each group had 10 nude mice models.

Application of the above Se atom doped water-soluble carbon quantum dots as a new type of photosensitizer in anti-microbial material: 200 µL of mold phosphate buffered solution of 2×10⁵ cfu/mL was added to sterile 24-well plates, then 10 µL of Se atom doped carbon quantum dots solution with a concentration of 0.3 mg/mL was added. The mixed solution was shaken and cultured for 0.5 hours in dark condition. After irradiation for 10 minutes with simulated sunlight of 400-800 nm wavelength at light intensity of 100 mW/cm², the mixed solution in 24-well plates was transferred to an agar plate with culture medium, and the survival rate of mold was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination.

The structural formula of polymer PT3 was as follows:

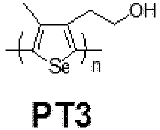

### Example 10

A method for preparing S, Si two-atom doped water-soluble carbon quantum dots, comprising the steps of:
adding 10 mg solid powder of polymer PT4 into a beaker, adding 40 mL of aqueous solution of potassium hydroxide with a concentration of 0.5 M, and mixing uniformly; transferring the mixed reaction solution into microwave reactor, keeping the reaction temperature at 250 °C for 12 hours, after cooling, separating and purifying to obtain S, Si two-atom doped water-soluble carbon quantum dots.

An application of the above S, Si two-atom doped water-soluble carbon quantum dots as a new type of photocatalyst in the degradation of organic pollutants in the environment: 100 mg of S, Si two-atom doped water-soluble carbon quantum dots was diffused into 100 mL of aqueous solution of rhodamine B of 10⁻⁵ M, and then the mixed solution was transferred into a sealable quartz vessel with a condensation device, stirred for 2 hours; the mixed solution was irradiated with 450W xenon lamp of 400-800 nm wavelength at light energy of 100 mW/cm²; 2 mL of solution was taken out at intervals of 2 minutes to measure the absorbance of rhodamine B at 553 nm by UV-Vis spectrometer.

An application of the above S, Si two-atom doped water-soluble carbon quantum dots as a new type of photocatalyst in the photocatalytic water-splitting for hydrogen generation: 150 mg of S, Si two-atom doped water-soluble carbon quantum dots were diffused into 100 mL of aqueous solution containing 10 wt% sodium sulfite, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 700 mW/cm² for 180 minutes, and the generated hydrogen was on-line analyzed by gas chromatography.

An application of the above S, Si two-atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated onto the transparent glass of indium tin oxide (ITO) with a thickness about 30 nm to form a hole transport auxiliary layer. Poly-3-hexylthiophene (P3HT) and S, Si two-atom doped carbon quantum dots were dissolved in the chlorobenzene solution by weight ratio of 10: 1, and was spin-coated in 2000 rpm onto the hole transport auxiliary layer to form an active layer of 70-90 nm thickness; finally an Al electrode was evaporated with a vacuum evaporation deposition machine, with annealing at 140 °C for 10 minutes to obtain organic polymer solar cell constructed by S, Si two-atom doped carbon quantum dots as a new type of electron acceptor material. The volt-ampere (I-V) characteristics of the solar cell both under illumination and in dark condition were measured.

An application of the above S, Si two-atom doped water-soluble carbon quantum dots as a new type of photosensitizer in quantum dot-sensitized solar cell: zinc oxide nanorods and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on the FTO conductive glass was heated up to 500°C, keeping the temperature for 120 minutes, to remove the organics in the film. The conductive glass electrode sintered at 500°C was cooled to 80 °C, and immersed into the S, Si two-atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the cell both under illumination and in dark condition were measured.

An application of the above S, N two-atom doped water-soluble carbon quantum dots as a new type of photosensitizer in the in vitro imaging and photodynamic therapy: the model for in vitro photodynamic therapy was SN12C kidney cancer cells. In dark condition, the SN12C kidney cancer cells and S, Si two-atom doped water-soluble carbon quantum dots with a concentration of 200 µg/mL were incubated in cell culture solution for 24 hours. After washing twice with PBS buffered solution, the labeling effect of cells was observed under a confocal microscope. Next, after irradiation with visible light of 400-800 nm wavelength at light intensity of 100 mW/cm² for 20 minutes, these cells were continued to be incubated in cell culture incubator for 48 hours. The survival rate of SN12C kidney cancer cells was detected by microplate reader.

An application of the above S, Si two-atom doped water-soluble carbon quantum dots as a new type of photosensitizer in the in vivo imaging and photodynamic therapy: the model for in vivo photodynamic therapy was nude mice subcutaneously inoculated with SN12C kidney cancer cells. When the SN12C kidney cancer tumors grew up to 30-35 mm³, 100 µL of S, Si two-atom doped water-soluble carbon quantum dots with a concentration of 5 mg/mL were injected into the tumors by subcutaneous injection. One hour later, the in vivo imaging and labeling effect was observed by in vivo imaging system. Next, the tumors were irradiated with visible light of 400-800 nm wavelength at light intensity of 100mW/cm² for 15 minutes, once daily for two days. The photographs of the nude mice and tumors after the treatment of photodynamic therapy were collected by a digital camera, and the tumor sizes were measured by vernier caliper. Two groups of comparative tests were used: one group of mice were injected with physiological saline only to let the tumor grow naturally; the other group of mice were injected with S, Si two-atom doped water-soluble carbon quantum dots only, without illumination. Each group had 10 nude mice models.

Application of the above S, Si two-atom doped water-soluble carbon quantum dots as a new type of photosensitizer in anti-microbial material: 200 µL of staphylococcus aureus phosphate buffered solution of 2× 10⁵ cfu/mL was added to sterile 24-well plates, then 10 µL of S, Si two-atom doped carbon quantum dots solution with a concentration of 0.5 mg/mL was added. The mixed solution was shaken and cultured for 0.5 hours in dark condition. After irradiation for 10 minutes with simulated sunlight of 400-800 nm wavelength at light intensity of 200 mW/cm², the mixed solution in 24-well plates was transferred to an agar plate with culture medium, and the survival rate of staphylococcus aureus was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination. The structural formula of polymer PT4 was as follows:

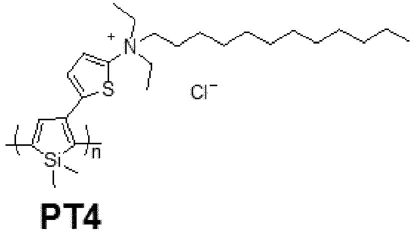

### Example 11

A method for preparing Se, N and P three-atom doped water-soluble carbon quantum dots, comprising the steps of:
adding mixed solid powders consisted of 10 mg of polymer PT3 and 10 mg of polymer PPV1 into a beaker, adding 40 mL of aqueous solution of sodium hydroxide with a concentration of 0.5 M, and mixing uniformly; transferring the mixed reaction solution into round flask, heating with oil bath, keeping the reaction temperature at 250 °C for 12 hours, and after cooling, separating and purifying to obtain Se, N and P three-atom doped water-soluble carbon quantum dots.

An application of the above Se, N and P three-atom water-soluble carbon quantum dots as a new type of photocatalyst in the degradation of organic pollutants in the environment: 100 mg of Se, N and P three-atom doped water-soluble carbon quantum dots were diffused into 100 mL of aqueous solution of methylene blue with a concentration of 10⁻⁵M, then the mixed solution was transferred into a sealable quartz vessel with a condensation device, stirred for 2 hours; the mixed solution was irradiated with 450W xenon lamp of 400-800 nm wavelength at light energy of 600 mW/cm²; 2 mL of solution was taken out at intervals of 2 minutes to measure the absorbance of methylene blue at 650 nm by UV-Vis spectrometer.

An application of the above Se, N and P three-atom doped water-soluble carbon quantum dots as a new type of photocatalyst in the photocatalytic water-splitting for hydrogen generation: 200 mg of Se, N and P three-atom doped water-soluble carbon quantum dots were diffused into 100 mL of aqueous solution containing 10 wt% lactic acid, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 1200 mW/cm² for 180 minutes, and the generated hydrogen was on-line analyzed by gas chromatography.

An application of the above Se, N and P three-atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated onto the transparent glass of indium tin oxide (ITO) with a thickness about 30 nm to form a hole transport auxiliary layer. Poly-3-hexylthiophene (P3HT) and Se, N, P three-atom doped carbon quantum dots were dissolved in the chlorobenzene solution by weight ratio of 10: 1, and was spin-coated in 2000 rpm onto the hole transport auxiliary layer to form an active layer of 70-90 nm thickness; finally an Al electrode was evaporated with a vacuum evaporation deposition machine, with annealing at 140 °C for 10 minutes to obtain organic polymer solar cell constructed by Se, N and P atoms doped carbon quantum dots as a new type of electron acceptor material. The volt-ampere (I-V) characteristics of the solar cell both under illumination and in dark condition were measured.

An application of the above Se, N and P three-atom doped water-soluble carbon quantum dots as a new type of photosensitizer in quantum dot-sensitized solar cell: titanium dioxide nanorods, and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on the FTO conductive glass was heated up to 500°C, keeping the temperature for 120 minutes, to remove the organics in the film. The conductive glass electrode sintered at 500°C was cooled to 80 °C, and immersed into the Se, N and P three-atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the solar cell both under illumination and in dark condition were measured.

An application of the above Se, N and P three-atom doped water-soluble carbon quantum dots as a new type of photosensitizer in the in vitro imaging and photodynamic therapy: the models for in vitro photodynamic therapy were TOV21 ovarian cancer cells. In dark condition, the TOV21 ovarian cancer cells and the Se, N and P three-atom doped water-soluble carbon quantum dots with a concentration of 100 µg/mL were incubated in cell culture solution for 4 hours. After washing twice with PBS buffered solution, the labeling effect of cells was observed under a confocal microscope. Next, the cancer cells were irradiated with visible light of 400-800 nm wavelength at light intensity of 50 mW/cm² for 20 minutes. The cancer cells were continued to be incubated in cell culture incubator for 48 hours. The survival rate of TOV21 ovarian cancer cells was detected by microplate reader.

An application of the above Se, N and P three-atom doped water-soluble carbon quantum dots as a new type of photosensitizer in the in vivo imaging and photodynamic therapy: the models for in vivo photodynamic therapy were nude mice subcutaneously inoculated with TOV21 ovarian cancer cells. When the TOV21 ovarian cancer tumors grew up to 30-35 mm³, 50 µL of Se, N and P three-atom doped water-soluble carbon quantum dots with a concentration of 8 mg/mL were injected into the tumors by subcutaneous injection. 2 hours later, the in vivo imaging and labeling effect was observed by in vivo imaging system. Next, the tumors were irradiated for 20 minutes with visible light of 400-800 nm wavelength at light intensity of 120mW/cm², once daily for two days. The photographs of the nude mice and tumors after the treatment of photodynamic therapy were collected by a digital camera, and the tumor sizes were measured by vernier caliper. Two groups of comparative tests were used: one group of mice were injected with physiological saline only to let the tumors grow naturally; the other group of mice were injected with Se, N and P three-atom doped water-soluble carbon quantum dots only, without illumination. Each group had 10 nude mice models.

Application of the above Se, N and P three-atom doped water-soluble carbon quantum dots as a new type of photosensitizer in anti-microbial material: 200 µL of bacteriophage phosphate buffered solution of 2× 10⁵ cfu/mL was added to sterile 24-well plates, then 10 µL of Se, N and P three-atom doped carbon quantum dots solution with a concentration of 1.5 mg/mL was added. The mixed solution was shaken and cultured for 0.5 hours in dark condition. After irradiation with simulated sunlight of 400-800 nm wavelength at light intensity of 120 mW/cm² for 10 minutes, the mixed solution in 24-well plates was transferred to an agar plate with culture medium, and the survival rate of bacteriophage was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination. The structural formulas of polymer PPV1 and PT3 were as follows:

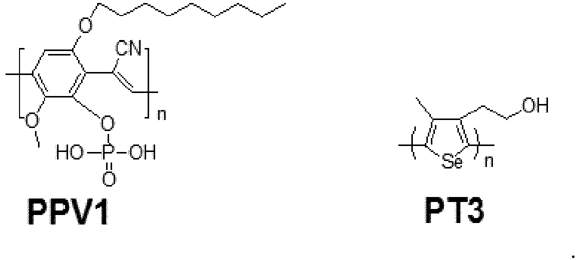

### Comparative Example

A method for preparing S, As two-atom doped water-soluble carbon quantum dots, comprising the steps of:
adding 10 mg solid powder of polymer PT7 into a beaker, adding 40 mL of aqueous solution of sodium hydroxide with a concentration of 0.5 M, and mixing uniformly; transferring the mixed reaction solution into hydrothermal reaction kettle, keeping the reaction temperature at 180 °C and the reaction time for 24 hours, after cooling, separating and purifying to obtain the S, As two-atom doped water-soluble fluorescence carbon quantum dots.

An application of the above S, As two-atom doped water-soluble carbon quantum dots as a new type of photocatalyst in the degradation of organic pollutants in the environment: 100 mg of As, S two-atom doped water-soluble carbon quantum dots was diffused into 100 mL of aqueous solution of rhodamine B with a concentration of 10⁻⁵ M, and then the mixed solution was transferred into a sealable quartz vessel with a condensation device, stirred for 2 hours; the mixed solution was irradiated with 450W xenon lamp of 400-800 nm wavelength at light energy of 400 mW/cm²; 2 mL of solution was taken out at intervals of 2 minutes to measure the absorbance of rhodamine B at 553 nm by UV-Vis spectrometer.

An application of the above S, As two-atom doped water-soluble carbon quantum dots as a new type of photocatalyst in the photocatalytic water-splitting for hydrogen generation: 50 mg of As, S two-atom doped water-soluble carbon quantum dots were diffused into 100 mL of aqueous solution containing 10 wt% triethanolamine, the mixed solution was transferred into a sealable quartz vessel with a condensation device, and high purity nitrogen was introduced to remove the dissolved oxygen in the water completely. Then, the mixed solution was irradiated with 450W xenon lamp of 400-800 nm wavelength at energy of 800 mW/cm² for 180 minutes, and the generated hydrogen was on-line analyzed by gas chromatography.

An application of the above S, As two-atom doped water-soluble carbon quantum dots as a new type of electron acceptor/donor material in construction of organic polymer solar cell: the conductive polymer polyethylenedioxythiophene (PEDOT) was mixed with polystyrene sulfonate (PSS) by weight ratio of 1: 25, then was spin-coated onto the transparent glass of indium tin oxide (ITO) with a thickness about 30 nm to form a hole transport auxiliary layer. Poly-3-hexylthiophene (P3HT) and S, As two-atom doped carbon quantum dots were dissolved in the chlorobenzene solution by weight ratio of 10: 1, and was spin-coated in 2000 rpm onto the hole transport auxiliary layer to form an active layer of 70-90 nm thickness; finally an Al electrode was evaporated with a vacuum evaporation deposition machine, with annealing at 140 °C for 10 minutes to obtain organic polymer solar cell constructed by S, As two-atom doped carbon quantum dots as a new type of electron acceptor material. The volt-ampere (I-V) characteristics of the solar cell both under illumination and in dark condition were measured.

An application of the above S, As two-atom doped water-soluble carbon quantum dots as a new type of photosensitizer in quantum dot- sensitized solar cell: titanium dioxide nanorod arrays and polyethylene glycol 20000 were mixed with water by weight ratio of 25: 10: 65 to obtain a homogeneous white viscous slurry, and the slurry was spin-coated onto the clean surface of the FTO conductive glass to form a titanium dioxide film. The titanium dioxide film on the FTO conductive glass was heated up to 500°C, keeping the temperature for 120 minutes, to remove the organics in the film. The conductive glass electrode sintered at 500°C was cooled to 80 °C, and immersed into the S, As two-atom doped carbon quantum dots aqueous solution with a concentration of 50 mg/mL, soaking for 48 hours at room temperature and in dark condition, then the electrode was taken out and assembled with a platinum electrode prepared by thermal evaporation into a cell. Electrolyte was added dropwise to the cell to complete a whole cell. The volt-ampere (I-V) characteristics of the solar cell both under illumination and in dark condition were measured.

An application of S, As two-atom doped water-soluble carbon quantum dots as a new type of photosensitizer in the in vitro imaging and photodynamic therapy: the models for in vitro photodynamic therapy were HT29 colon cancer cells. In dark condition, the HT29 colon cancer cells and S, As two-atom doped water-soluble carbon quantum dots with a concentration of 100 µg/mL were incubated in cell culture solution for 4 hours. After washing twice with PBS buffered solution, the labeling effect of cells was observed under a confocal microscope. Next, these cells were irradiated with visible light of 400-800 nm wavelength at light intensity of 50 mW/cm² for 20 minutes. Then these cells were continued to be incubated in cell culture incubator for 48 hours. The survival rate of HT29 colon cancer cells was detected by microplate reader.

An application of S, As two-atom doped water-soluble carbon quantum dots as a new type of photosensitizer in the in vivo imaging and photodynamic therapy: the models for in vivo photodynamic therapy were nude mice subcutaneously inoculated with HT29 colon cancer cells. When the HT29 colon cancer tumors grew up to 30-35 mm³, 50 µL of S, As two-atom doped water-soluble carbon quantum dots with a concentration of 5 mg/mL were injected into the tumors by subcutaneous injection. 2 hours later, the in vivo imaging and labeling effect was observed by in vivo imaging system. Next, the tumors were irradiated with visible light of 400-800 nm wavelength at light intensity of 150 mW/cm² for 20 minutes, once daily for two days. The photographs of the nude mice and tumors after treatment of photodynamic therapy were collected by a digital camera, and the tumor sizes were measured by vernier caliper. Two groups of comparative tests were used: one group of mice were injected with physiological saline only to let the tumor grow naturally; the other group of mice were injected with S, As two-atom doped water-soluble carbon quantum dots only, without illumination. Each group had 10 nude mice models.

Application of the above S, As two-atom doped water-soluble carbon quantum dots as a new type of photosensitizer in anti-microbial material: 200 µL of tobacco mosaic virus hydrochloric acid buffered solution with a concentration of 2×10¹¹ pfu/mL was added to sterile 24-well plates, then 10 µL of As, S two-atom doped carbon quantum dots solution with a concentration of 1.0 mg/mL was added. The mixed solution was shaken and cultured for 0.5 hours in dark condition. After irradiation for 10 minutes with simulated sunlight of 400-800 nm wavelength at light intensity of 150 mW/cm², the mixed solution in 24-well plates was transferred to an agar plate with culture medium, and the survival rate of tobacco mosaic virus was calculated by colony counting method. In addition, two groups of comparative tests were used: for one group the phosphate buffered solution being mixed with bacterium suspension, without illumination; for the other group the phosphate buffered solution and aqueous solution of carbon quantum dots were mixed with bacterium suspension, without illumination. The structural formula of polymer PT7 was as follows:

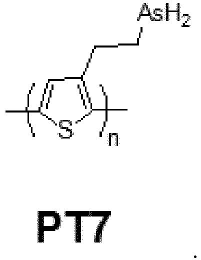

## Claims

1. A method of preparing heteroatom doped multifunctional carbon quantum dots from a precursor comprising a conjugated polymer having at least one heteroatom selected from the group consisting of O, N, S, Si, Se, P, As, Ge, Gd, B, Sb and Te with different functional groups on the surface thereof, wherein the method comprises the steps of:
1) adding to the conjugated polymer, 0-1 M aqueous solution of acids or bases with the mass of 0.01-1000 times as many as the mass of the conjugated polymer, mixing uniformly and obtaining a reaction solution;
2) heating the reaction solution up to 100 °C -500 °C, and reacting for 1-48 hours;
3) cooling after the reaction, collecting the reaction solution, separating and purifying to obtain heteroatom doped multifunctional carbon quantum dots;
wherein the conjugated polymer is one or more selected from the group consisting of the conjugated polymers with following structural formula: 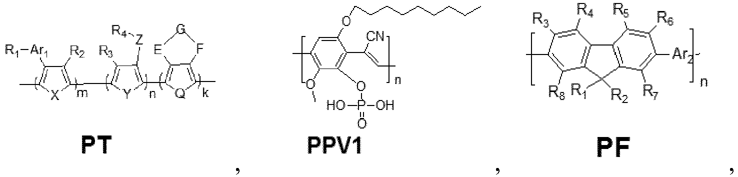 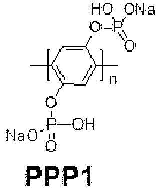 wherein:
in the structural formula of PT, m, n and k are natural numbers in range of 0-10000, while m, n and k do not represent 0 simultaneously;
in the structural formulas of PPV1, PF and PPP1, n is a natural number in range of 1-10000;
wherein: Ar₁ is furan, thiophene, selenophene, pyrrole, pyridine, benzene, naphthalene, anthracene, pyrene, indole, coumarin, fluorescein, carbazole, rhodamine, cyano dyes, fluorene or quinoline;
wherein: Ar₂ is one of following structural formulas: 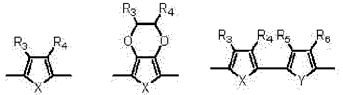 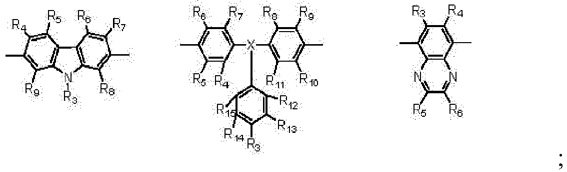
wherein: X, Y, Q, E and F respectively or simultaneously independently represent O, N, S, Si, Se, P, As, Ge, Gd, B, Sb, Te, N-R₅ or Si-R₆R₇;
wherein: Z, G, R₁, R₂, R₃, R₄, R₅, R₆, R₇, R₈, R₉, R₁₀, R₁₁, R₁₂, R₁₃, R₁₄ and R₁₅ respectively or simultaneously independently represent hydrogen atom, alkyl group of 1-18 carbon atoms, hydroxyl group, mercapto group, carboxyl group, amino group, amide, acid anhydride, cyano group, alkenyl, alkynyl, aryl group, ester group, ether group, quaternary ammonium salt, sulfonate, phosphate or polyethylene glycol group.

2. The method according to claim 1, wherein, in step 1), the acid is one or more selected from the group consisting of hydrochloric acid, hypochlorous acid, perchloric acid, hydrobromic acid, hypobromous acid, hyperbromic acid, iodic acid, hypoiodous acid, periodic acid, hydrofluoric acid, boric acid, nitric acid, nitrous acid, acetic acid, citric acid, sulfuric acid, sulfoxylic acid, carbonic acid, phosphoric acid, pyrophosphoric acid and hypophosphorous acid.

3. The method according to claim 1, wherein in step 1), the base is one or more selected from the group consisting of alkali metal hydroxide, alkaline earth metal hydroxide, phosphate, hydrogen phosphate, dihydrogen phosphate and ammonia.

4. The method according to claim 1, wherein in step 2), the reaction solution is heated with oil bath, in microwave reactor, ultrasonic reactor or hydrothermal reaction kettle.

## Patentansprüche

1. Verfahren zur Herstellung von heteroatomdotierten multifunktionellen Kohlenstoff-Quantenpunkten aus einem Vorläufer, umfassend ein konjugiertes Polymer mit mindestens einem Heteroatom, ausgewählt aus der Gruppe bestehend aus O, N, S, Si, Se, P, As, Ge, Gd, B, Sb und Te mit unterschiedlichen funktionellen Gruppen auf der Oberfläche davon, wobei das Verfahren die Schritte umfasst:
1) Zugeben von 0-1 M wässriger Lösung von Säuren oder Basen zu dem konjugierten Polymer, mit einer Masse, die 0,01-1000 mal so groß ist wie die Masse des konjugierten Polymers, gleichmäßiges Mischen und Erhalten einer Reaktionslösung;
2) Erhitzen der Reaktionslösung auf bis zu 100 °C-500 °C und Umsetzen für 1-48 Stunden;
3) Abkühlen nach der Umsetzung, Sammeln der Reaktionslösung, Trennen und Reinigen, um heteroatomdotierte multifunktionelle Kohlenstoff-Quantenpunkte zu erhalten;
wobei das konjugierte Polymer eines oder mehrere sind, die ausgewählt sind aus der Gruppe bestehend aus den konjugierten Polymeren mit folgender Strukturformel: 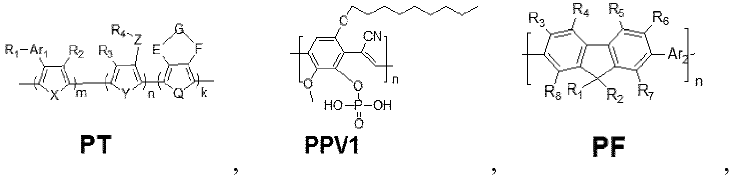 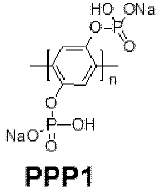 wobei:
in der Strukturformel von PT m, n und k natürliche Zahlen im Bereich von 0-10000 sind, während m, n und k nicht gleichzeitig 0 darstellen;
in den Strukturformeln von PPV1, PF und PPP1 n eine natürliche Zahl im Bereich von 1-10000 ist;
wobei: Ar₁ Furan, Thiophen, Selenophen, Pyrrol, Pyridin, Benzol, Naphthalin, Anthracen, Pyren, Indol, Cumarin, Fluorescein, Carbazol, Rhodamin, Cyano-Farbstoffe, Fluoren oder Chinolin ist;
wobei: Ar₂ eine von folgenden Strukturformeln ist: 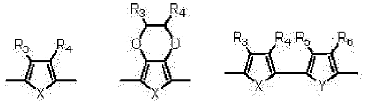 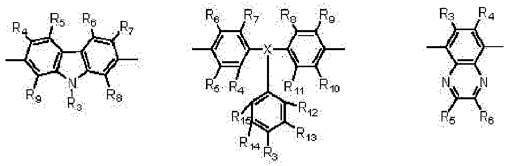
wobei: X, Y, Q, E und F jeweils oder gleichzeitig, unabhängig voneinander O, N, S, Si, Se, P, As, Ge, Gd, B, Sb, Te, N-R₅ oder Si-R₆R₇ darstellen;
wobei: Z, G, R₁, R₂, R₃, R₄, R₅, R₆, R₇, R₈, R₉, R₁₀, R₁₁, R₁₂, R₁₃, R₁₄ und R₁₅ jeweils oder gleichzeitig, unabhängig voneinander ein Wasserstoffatom, eine Alkylgruppe von 1-18 Kohlenstoffatomen, eine Hydroxylgruppe, Mercaptogruppe, Carboxylgruppe, Aminogruppe, ein Amid, Säureanhydrid, eine Cyanogruppe, Alkenyl-, Alkinyl-, Arylgruppe, Estergruppe, Ethergruppe, ein quaternäres Ammoniumsalz, Sulfonat, Phosphat oder eine Polyethylenglykolgruppe darstellen.

2. Verfahren nach Anspruch 1, wobei in Schritt 1) die Säure eine oder mehrere sind, die ausgewählt sind aus der Gruppe bestehend aus Salzsäure, Hypochlorsäure, Perchlorsäure, Bromwasserstoffsäure, hypobromiger Säure, Hyperbromsäure, Iodsäure, hypoiodige Säure, Periodsäure, Flusssäure, Borsäure, Salpetersäure, salpetrige Säure, Essigsäure, Zitronensäure, Schwefelsäure, Sulfoxylsäure, Kohlensäure, Phosphorsäure, Pyrophosphorsäure und Phosphinsäure.

3. Verfahren nach Anspruch 1, wobei in Schritt 1) die Base eine oder mehrere sind, die ausgewählt sind aus der Gruppe bestehend aus Alkalimetallhydroxid, Erdalkalimetallhydroxid, Phosphat, Hydrogenphosphat, Dihydrogenphosphat und Ammoniak.

4. Verfahren nach Anspruch 1, wobei in Schritt 2) die Reaktionslösung mit einem Ölbad in einem Mikrowellenreaktor, einem Ultraschallreaktor oder einem hydrothermalen Reaktionskessel erhitzt wird.

## Revendications

1. Procédé de préparation de points quantiques de carbone multifonctionnel dopés par des hétéroatomes depuis un précurseur comprenant un polymère conjugué présentant au moins un hétéroatome sélectionné à partir du groupe constitué par O, N, S, Si, Se, P, As, Ge, Gd, B, Sb et Te avec différents groupes fonctionnels sur la surface de celui-ci, dans lequel le procédé comprend les étapes consistant à :
1) ajouter au polymère conjugué, une solution aqueuse d'acides ou de bases de 0 à 1 M avec la masse de 0,01 à 1000 fois aussi grande que la masse du polymère conjugué, mélanger uniformément et obtenir une solution de réaction ;
2) chauffer la solution de réaction jusqu'à de 100 °C à 500 °C, et faire réagir pendant de 1 à 48 heures ;
3) faire refroidir après la réaction, collecter la solution de réaction, séparer et purifier pour obtenir des points quantiques de carbone multifonctionnel dopés par des hétéroatomes;
dans lequel le polymère conjugué est un ou plusieurs sélectionnés à partir du groupe constitué par les polymères conjugués avec les formules structurelles suivantes : 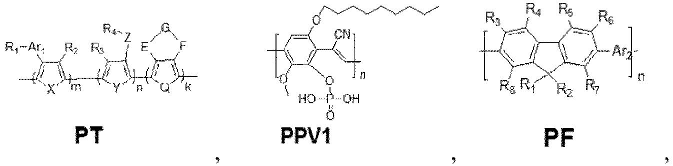 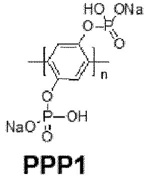 dans lequel :
dans la formule structurelle de PT, m, n et k sont des nombres naturels dans la plage de 0 à 10 000, tandis que m, n et k ne représentent pas 0 simultanément ;
dans les formules structurelles de PPV1, PF et PPP1, n est un nombre naturel dans la plage de 1 à 10 000;
dans lequel : Ar₁ est le furane, le thiophène, le sélènophène, le pyrrole, la pyridine, le benzène, le naphtalène, l'anthracène, le pyrène, l'indole, la coumarine, la fluorescéine, le carbazole, la rhodamine, des colorants cyano, le fluorène ou la quinoléine ;
dans lequel : Ar₂ est une des formules structurelles suivantes : 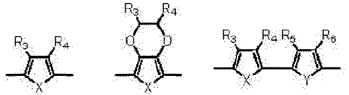 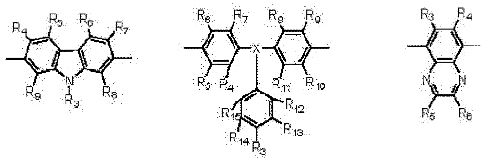
dans lequel : X, Y, Q, E et F représentent respectivement ou simultanément indépendamment O, N, S, Si, Se, P, As, Ge, Gd, B, Sb, Te, N-R₅ ou Si-R₆R₇ ;
dans lequel : Z, G, R₁, R₂, R₃, R₄, R₅, R₆, R₇, R₈, R₉, R₁₀, R₁₁, R₁₂, R₁₃, R₁₄ et R₁₅ représentent respectivement ou simultanément indépendamment un atome d'hydrogène, un groupe alkyle de 1 à 18 atomes de carbone, un groupe hydroxyle, un groupe mercapto, un groupe carboxyle, une groupe amino, un amide, un anhydride d'acide, un groupe cyano, un alcényle, un alcynyle, un groupe aryle, un groupe ester, un groupe éther, un sel d'ammonium quaternaire, un sulfonate, un phosphate ou un groupe polyéthylène glycol.

2. Procédé selon la revendication 1, dans lequel, à l'étape 1), l'acide est un ou plusieurs sélectionnés à partir du groupe constitué par l'acide hydrochlorique, l'acide hypochloreux, l'acide perchlorique, l'acide bromhydrique, l'acide hypobromeux, l'acide hyperbromique, l'acide iodique, l'acide hypoiodeux, l'acide périodique, l'acide fluorhydrique, l'acide borique, l'acide nitrique, l'acide nitreux, l'acide acétique, l'acide citrique, l'acide sulfurique, l'acide sulfoxylique, l'acide carbonique, l'acide phosphorique, l'acide pyrophosphorique et l'acide hypophosphoreux.

3. Procédé selon la revendication 1, dans lequel, à l'étape 1), la base est un ou plusieurs sélectionnés à partir du groupe constitué par l'hydroxyde de métal alcalin, l'hydroxyde de métal alcalino-terreux, le phosphate, le phosphate d'hydrogène, le phosphate de dihydrogène et l'ammoniac.

4. Procédé selon la revendication 1, dans lequel, à l'étape 2), la solution de réaction est chauffée avec un bain d'huile, dans un réacteur à micro-ondes, un réacteur ultrasonique ou une bouilloire à réaction hydrothermique.
